# EUROPÄISCHE PATENTSCHRIFT

(11) **EP 1 638 648 B1**
(45) Veröffentlichungstag und Bekanntmachung des Hinweises auf die Patenterteilung: **17.12.2008**
(21) Anmeldenummer: 04738488.8
(22) Anmeldetag: 07.05.2004
(51) Int. Cl.: A61N 5/067, A61H 39/00

(54) **VORRICHTUNG ZUR AKUPUNKTUR MITTELS LASERSTRAHLUNG**
ACUPUNCTURE DEVICE USING A LASER BEAM
DISPOSITIF D'ACUPONCTURE FAISANT APPEL A UN RAYON LASER

(30) Priorität: 27.06.2003 DE 20309976 U
(43) Veröffentlichungstag der Anmeldung: 29.03.2006
(73) Patentinhaber: Weber, Michael, 37697 Lauenförde (DE)
(72) Erfinder: Weber, Michael, 37697 Lauenförde (DE)
(74) Vertreter: Seewald, Jürgen
(86) Internationale Anmeldenummer: PCT/DE2004/000960
(87) Internationale Veröffentlichungsnummer: WO 2005/009538

(56) Entgegenhaltungen:
- EP-A- 0 495 757
- WO-A-02/40098
- DE-A- 2 740 969
- DE-U- 9 105 621
- DE-U- 29 519 433
- US-A- 6 074 411

## Beschreibung

Die Erfindung betrifft eine Vorrichtung zur Akupunktur mittels Laserstrahlung.

Aus der WO 02/40098 A1 ist eine Vorrichtung zur Akupunktur mittels Laserstrahlung bekannt, welche eine Einrichtung zur Erzeugung der Laserstrahlung und ein damit verbundenes Handstück aufweist. Das Handstück ist mit einer mit der Laserstrahlerzeugungseinrichtung verbundenen Lichtleitfaser versehen, welche bei der Akupunktur in Kontakt mit dem Körper eines Patienten steht und die Laserstrahlung auf denselben aufbringt bzw. für das Eindringen der Laserstrahlung in den Körper sorgt.

Die Wirksamkeit dieser Vorrichtung bei der Schmerzbehandlung steht außer Frage, es hat sich allerdings herausgestellt, dass die bisher aus handelsüblichen Kunststofffasern hergestellten Lichtleitfasern, welche die Laserstrahlung von der Laserstrahlerzeugungseinrichtung bis zu dem Ausgangsbereich jedes einzelnen Handstücks transportieren, der dauerhaften Belastung beim praktischen Einsatz dieser Vorrichtungen unter Umständen nicht gewachsen sind und teilweise brechen bzw. Leistung verlieren. Ein weiteres Problem dieser bekannten Vorrichtung ist die Tatsache, dass die den Ausgangsbereich der Lichtleitfaser bildende Spitze bei den herkömmlichen Kunststoffmaterialien verhältnismäßig schnell stumpf wird, insbesondere da sich Kosmetika, welche sich auf der Haut der Patienten befinden, in die Spitze einbrennen können. Weiterhin können die Makromolekülstrukturen der Kunststofffasern durch die permanente Beanspruchung denaturieren und degenerieren und so einen Leistungsverlust der transportierten Laserenergie nach sich ziehen.

Die US 6,074,411 beschreibt eine Laservorrichtung und ein Laserverfahren, mit denen eine Akupunktur am Körper eines Patienten durchführbar ist. Dabei sind einzelne Laserdioden mittels elektrischer Leitungen an einem Steuergerät angeschlossen. Die Laserdioden sind innerhalb eines Gehäuses angeordnet, welches auf den Körper des Patienten aufgeklebt wird.

Mit derartigen Vorrichtungen kann allerdings die gewünschte Wirkung bei der Schmerztherapie mittels Akupunktur bei weitem nicht erreicht werden, da die Laserstrahlung aufgrund der großflächigen Bestrahlung nur in die äußersten Hautschichten eindringen kann.

Die DE 90 10 925 U1 beschreibt eine Vorrichtung zum Bestrahlen von Blut, bei welcher das Blut durch ein Glasrohr geleitet wird, welches einer UV-Bestrahlung ausgesetzt ist.

Die DE 91 05 621 U1 offenbart eine Vorrichtung zur Akupunktur mittels Laserstrahlung, wobei die Vorrichtung ein Steuergerät, welches mehrere Ausgänge für Stromleitungen aufweist, mehrere Stromleitungen, welche an die Ausgänge des Steuergeräts angeschlossen sind und mehrere Laserdioden, welche jeweils an die Stromleitungen angeschlossen sind, aufweist. Die Laserdioden sind jeweils an einer Halterung befestigt. Die Halterung umfasst zusätzlich eine Haftvorrichtung, mit dem die Halterung mit der Laserdiode an einer Haut befestigt werden kann.

Es ist Aufgabe der vorliegenden Erfindung, eine Vorrichtung zur Akupunktur mittels Laserstrahlung zu schaffen, welche einerseits in der Lage ist, eine für eine sinnvolle Schmerzbehandlung eines Patienten angemessene Wirkung hervorzurufen, welche jedoch andererseits für sämtliche Gesichtspunkte des praktischen Einsatzes, insbesondere bezüglich einer leichten Austauschbarkeit der Laserdioden bzw. Lasermodule mit verschiedenen Wellenlängen und Diodenstärken und bezüglich einer langen Lebensdauer sämtlicher Elemente, geeignet ist.

Erfindungsgemäß wird diese Aufgabe durch die in Anspruch 1 genannten Merkmale gelöst.

Durch die einzelnen Glasfasernadeln bzw. Nadeln aus glasfaseranalogen Kunststofffasern (nachfolgend, auch in den Patentansprüchen, abkürzend "Lasernadeln" genannt), in welche die von den Laserdioden ausgesandte Laserstrahlung eingekoppelt wird, ist aufgrund der hierdurch gegebenen Fokussierung des Laserstrahls sowie dem direkten Kontakt der Lasernadeln mit dem Körper sichergestellt, dass die Laserstrahlung mit einer sehr hohen Energiedichte in den Körper des Patienten eingeleitet wird, so dass eine sehr hohe Effizienz der erfindungsgemäßen Vorrichtung sichergestellt ist. Durch die Verwendung von Glasfaser oder glasfaseranaloger Kunststofffaser ( nachfolgend, auch in den Patentansprüchen, abkürzend "Lichtleitfaser" genannt) für die Lasernadeln, welche mit dem Körper des Patienten in Verbindung treten, ist außerdem eine sehr hohe Langlebigkeit der Vorrichtung gegeben, da die Lasernadeln sehr unempfindlich gegenüber Beschädigungen aufgrund irgendwelcher Fremdkörper sind. Weiterhin sind die Laserdioden durch ihre Auslagerung aus dem Steuergerät für die Wartung, Reparatur bzw. den Austausch leicht zugänglich.

Die Stromleitungen sind im Bündel in oder an einem Haltearm von dem Steuergerät zu einer am Ende des Haltearms angeordneten Modulplatte geführt, in oder an der eine Vereinzelung der Stromleitungen des Bündels erfolgt, wobei an die Enden der Stromleitungen an der Modulplatte jeweils ein ein Treiberchip und eine Laserdiode aufweisendes Lasermodul angeschlossen ist und die Lasermodule jeweils über hängende, durch eine Umhüllung geschützte Lichtleitfasern mit den Lasernadeln verbunden sind. Dabei können die Lasernadeln einstükkig mit den Lichtleitfasern ausgebildet oder aber jeweils über ein optisches Koppelelement mit diesen verbunden sein. Auch wenn Lichtleitfasern zwischen den Laserdioden und den Lasernadeln angeordnet sind, wird die Laserstrahlung mit hoher Energiedichte in den Körper eines Patienten eingeleitet, da für die Laserstrahlung in der Lichtleitfaser optimale Leitungsbedingungen vorliegen. Ein weiterer Vorteil ergibt sich, wenn die Lasermodule im peripheren Bereich der Modulplatte angeordnet sind und im zentralen Bereich ein Lüfter angeordnet ist. Dadurch wird eine effektive Kühlung der Lasermodule erreicht, deren übermäßige Erwärmung bei den aus dem Stand der Technik bekannten Vorrichtungen sehr häufig zu Ausfällen führt.

In Weiterbildung der Erfindung ist es vorteilhaft, wenn die Stromleitungen mittels jeweiliger Steckverbindungen mit der jeweiligen Laserdiode bzw. Lasermodulen verbunden sind. Dadurch können die unterschiedlichen Lasernadeln und/oder die mit denselben verbundenen Laserdioden bzw. Lasermodule in sehr einfacher Weise ausgetauscht und somit an den jeweiligen Anwendungsfall angepasst werden, ohne dass spezielles Servicepersonal erforderlich ist. Der Benutzer kann sich auf diese Weise also einen individuellen Satz an Lasernadeln zusammenstellen. Im unwahrscheinlichen Fall von Verschleiß bzw. einem Defekt an einer der Lasernadeln ist es in einem solchen Fall des weiteren möglich, für einen sehr schnellen und unkomplizierten Austausch zu sorgen. Bei Anordnung von Lichtleitfasern_zwischen Lasermodulen und Lasernadeln ist auch eine Steckverbindung zwischen Lasermodul und Lichtleitfaser_sinnvoll, da diese ein Austausch der Lasernadeln ohne gleichzeitigen Wechsel der Lasermodule ermöglicht.

Da das Steuergerät lediglich Stromimpulse an die Laserdioden aussenden muss, kann es vorteilhafterweise sehr klein ausgeführt werden, so dass es zum einen für den mobilen Einsatz geeignet ist und zum anderen gegebenenfalls auch am Körper des zu behandelnden Patienten angebracht werden kann, so dass dieser sich mit dem Steuergerät bewegen kann. Dies ist vor allem beim Einsatz der erfindungsgemäßen Vorrichtung bei Kindern oder auch bei Tieren vorteilhaft.

Ein noch größerer Schutz der Lasernadeln gegen eventuelle Beschädigungen kann sich in einer vorteilhaften Weiterbildung der Erfindung dadurch ergeben, dass die Lasernadeln mit Kunststoffhülsen überzogen sind.

Wenn in einer weiteren Ausgestaltung der Erfindung die Lasernadeln und/oder ein Kern der Lasernadeln sich von der Laserdiode zu ihrem Ausgangsbereich verjüngen, so ergibt dies eine noch höhere Energiedichte im Ausgangsbereich der Lasernadeln, wodurch eine noch höhere Reizwirkung am Körper des Patienten erzielt werden kann. Alternativ ist es bei Lasernadeln mit einer derartigen Verjüngung jedoch selbstverständlich auch möglich, Laserdioden mit niedrigerer Leistung zu verwenden, um dieselbe Reizwirkung wie mit Lasernadeln ohne eine solche Verjüngung zu erreichen.

Eine sehr einfache Handhabung der Stromleitungen, insbesondere wenn dieselben defekt sein sollten und ausgetauscht werden müssen, ergibt sich, wenn die Stromleitungen mittels jeweiliger Steckverbindungen mit dem Steuergerät verbunden sind.

Für den mobilen Einsatz der erfindungsgemäßen Vorrichtung kann in einer weiteren vorteilhaften Ausgestaltung der Erfindung vorgesehen sein, dass das Steuergerät mittels einer Batterie betreibbar ist.

In einer weiteren Ausgestaltung der Erfindung kann vorgesehen sein, dass die Lasernadel von einer stromleitenden Hülse umgeben ist, welche von dem Steuergerät mit Strom versorgt wird. Durch eine derartige, mit Strom versorgte Hülse ist es möglich, den durch die Laserstrahlung verursachten Reiz im Körper des Patienten noch zu verstärken und somit bessere Ergebnisse bei der Schmerzbehandlung zu erzielen.

Weitere vorteilhafte Ausgestaltungen und Weiterbildungen der Erfindung ergeben sich aus den restlichen Unteransprüchen sowie aus den nachfolgend anhand der Zeichnung prinzipmäßig dargestellten Ausführungsbeispielen.

Es zeigt:
Fig. 1 eine erste nicht erfindungsgemäße Vorrichtung zur Akupunktur mittels Laserstrahlung;
Fig. 2 eine zweite nicht erfindungsgemäße Vorrichtung zur Akupunktur mittels Laserstrahlung,
(Fig. 3 und 4 wurden ersatzlos gestrichen)
Fig. 5 eine Ausführungsform der erfindungsgemäßen Vorrichtung,
Fig. 6 eine Einzelheit A gemäß Fig. 5 in vergrößerter Darstellung,
Fig. 7 eine Druntersicht der Einzelheit A gemäß Fig. 6,
Fig. 8 eine erste Möglichkeit der Fixierung einer Lasernadel am Körper eines Patienten,
Fig. 9 eine zweite Möglichkeit der Fixierung einer Lasernadel am Körper eines Patienten, und
Fig. 10 eine vergrößerte Darstellung einer eine Lasernadel umhüllenden Kunststoffhülse.

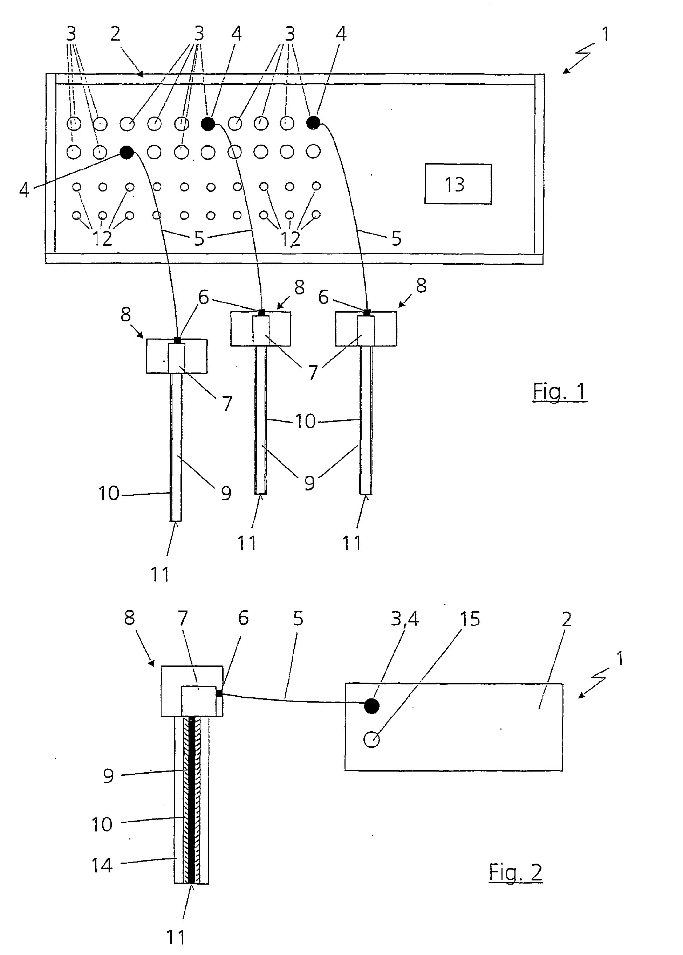

Fig. 1 zeigt eine schematische Darstellung einer Vorrichtung 1 zur Akupunktur eines nicht dargestellten Körperteils eines Patienten mittels Laserstrahlung. Die Vorrichtung 1 weist ein Steuergerät 2 auf, welches mit mehreren Ausgänge 3 versehen ist. Im vorliegenden Fall handelt es sich um insgesamt zwanzig Ausgänge 3, es kann jedoch auch jede andere Anzahl an Ausgängen 3 vorgesehen sein. An die Ausgänge 3 sind über jeweilige Steckverbindungen 4 jeweilige Stromleitungen 5 angeschlossen, welche von dem Steuergerät 2 mit einem Strom, insbesondere einem Schwachstrom, versorgt werden. Die an die Stromleitung 5 angelegte Spannung sowie die Stromstärke können mittels nicht dargestellter Regler an dem Steuergerät 2 verstellt werden.

An jede Stromleitung 5 ist wiederum über jeweilige Steckverbindungen 6 jeweils eine Laserdiode 7 angeschlossen, welche in der Lage ist, Laserstrahlung auszusenden. Die Verbindung der Laserdioden 7 mit der Stromleitung 5 kann auch durch Verlöten dieser beiden Teile erfolgen. Jede einzelne Laserdiode 7 ist in einem Handstück 8 untergebracht und mit einer jeweiligen Lasernadel 9 beispielsweise durch Verkleben verbunden. Im vorliegenden Ausführungsbeispiel sind insgesamt lediglich drei Handstücke 8 mit jeweiligen Lasernadeln 9, der zugehörigen Laserdiode 7 und der Stromleitung 5 dargestellt, es könnten jedoch an sämtliche Ausgänge 3 des Steuergeräts 2 entsprechende Lasernadeln 9 angeschlossen werden, um mehrere Akupunkturpunkte am Körper des Patienten gleichzeitig zu behandeln.

Die Lasernadeln 9 sind zu ihrem Schutz mit jeweiligen Kunststoffhülsen 10 umgeben und sind dafür vorgesehen, in ihrem jeweiligen Ausgangsbereich 11 mit dem Körper des Patienten in Kontakt zu treten. Die von den Laserdioden 7 ausgesandte Laserstrahlung wird durch die direkte Verbindung mit den Lasernadeln 9 in dieselben eingekoppelt und im wesentlichen ohne Verluste an den Körper des Patienten weitergeleitet.

Um eine Verstärkung der von den Laserdioden 7 ausgesandten Laserstrahlung zu erreichen, können sich die Lasernadeln 9 von der jeweiligen Laserdiode 7 zu ihrem Ausgangsbereich 11 in nicht dargestellter Weise verjüngen. Die Verjüngung der Lasernadeln 9 ist dabei proportional zu der Verstärkung der Laserstrahlung. In diesem Zusammenhang können die Laserdioden 7 unterschiedliche Leistungen, beispielsweise in einem Bereich von 50 bis 200 mW aufweisen. Neben der unterschiedlichen Leistung können die einzelnen Laserdioden 7 auch unterschiedliche Wellenlängen aufweisen, um für unterschiedliche Einsatzzwecke an ein und demselben Patienten geeignet zu sein. Des weiteren ist es möglich, die Frequenz der von den Laserdioden 7 ausgesandten Laserstrahlung mittels des Steuergeräts 2 zu modulieren, um eine gepulste Laserstrahlung zu erreichen und somit verschiedene Arten von Geweben reizen bzw. ansprechen zu können.

Das Steuergerät 2 weist für jeden Ausgang 3 eine Intensitätsanzeige 12 auf, die die Intensität der von den Laserdioden 7 ausgesandten Laserstrahlung in digitaler Form anzeigt. Des weiteren ist das Steuergerät 2 mit einem Photometer 13 versehen, welches dafür vorgesehen ist, die Intensität der Laserstrahlung in dem Ausgangsbereich 11 der Lasernadel 9 zu messen, beispielsweise um die ordnungsgemäße Funktion derselben zu prüfen. Hierfür muss die jeweilige Lasernadel 9 lediglich vor das Photometer 13 gehalten werden.

Das Steuergerät 2 kann eine derartige Größe aufweisen, dass es mittels nicht dargestellter Halteeinrichtungen am Körper der zu behandelnden Person befestigt werden kann. Um eine vollständige Unabhängigkeit von einem Stromnetz zu erreichen, kann das Steuergerät 2 des weiteren mittels einer ebenfalls nicht dargestellten Batterie betrieben werden. Des weiteren kann das Steuergerät 2 eine Zeitschaltuhr zur zeitlichen Steuerung desselben aufweisen.

Fig. 2 zeigt eine weitere Ausführungsform der Vorrichtung 1, bei der die Lasernadel 9 außer von der Kunststoffhülse 10 auch von einer Hülse 14 umgeben ist, welche über die jeweilige Stromleitung 5 mit Strom versorgt wird, um zusätzlich zur Reizung des Gewebes durch die Laserstrahlung auch eine Reizwirkung durch Strom zu erreichen und somit die Wirkung der Laserstrahlung zu verstärken. Die Kunststoffhülse 10 dient dabei einerseits zum Schutz der Lasernadel 9 und andererseits zur Isolierung derselben gegenüber der stromdurchflossenen Hülse 14. Die aus einem stromleitenden Material, z.B. Stahl oder Kupfer, bestehende Hülse 14 kann von einer weiteren, im vorliegenden Fall jedoch nicht dargestellten Kunststoffhülse umgeben sein, um eine weitere Isolierung zu erreichen.

Die stromdurchflossene Hülse 14 kann auch dazu verwendet werden, den Akupunkturpunkt durch elektrischen und eventuell zusätzlichen optischen Reiz zu stimulieren, um ihn damit mittels Pulsreflex zu lokalisieren. Dadurch kann eine genauere Bestimmung der Akupunkturpunkte erfolgen bzw. es können aktive Punkte sicherer aufgefunden werden. Des weiteren kann die stromdurchflossene Hülse 14 auch dazu benutzt werden, den Akupunkturpunkt mittels Hautwiderstandsmessung sicherer zu lokalisieren.

Prinzipiell wäre es auch möglich, zur Lokalisierung der Akupunkturpunkte an einen der Ausgänge 3 des Steuergeräts 2 eine an sich bekannte Diagnostiknadel anzuschließen, die keine Laserstrahlung erzeugt.

Das Steuergerät 2, von dem im vorliegenden Fall lediglich einer der Ausgänge 3 dargestellt ist, weist einen Regler 15 auf, mit dem die Intensität des der Hülse 14 zugeführten Stroms verstellt werden kann.

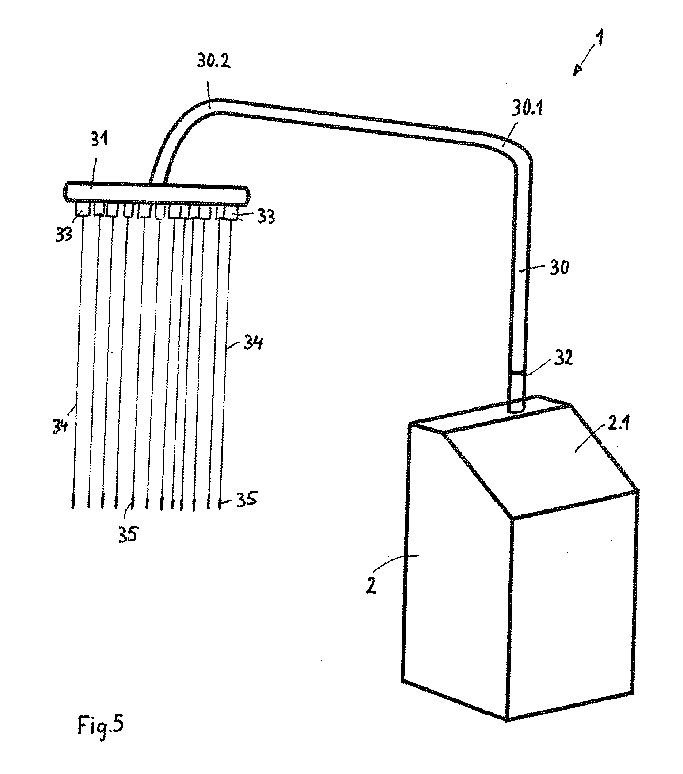

Die in Figur 5 gezeigte Vorrichtung 1 zur Akupunktur eines Patienten mittels Laserstrahlung besitzt ein Steuergerät 2, welches die gleichen Funktionen aufweist wie die im Zusammenhang mit den Figuren 1 und 2 erläuterten Steuergeräte. Das Gehäuse des Steuergerätes 2 besitzt ein Bedienpult 2.1. Von der Oberseite des Steuergerätes 2 ragt ein hohler Tragarm 30 nach oben ab, der in einem ersten Knie 30.1 in einen etwa horizontalen Bereich übergeht, und einem zweiten Knie 30.2 sich wieder abwärts neigt und in einer Modulplatte 31 endet. Der Haltearm 30 ist also insgesamt etwa galgenförmig ausgeführt. Er besitzt in seinem lotrechten Teil, kurz oberhalb des Steuergerätes 2 ein Drehgelenk 32, so dass der oberhalb des Drehgelenkes 32 befindliche Teil des Haltearms 30 gegenüber dem Steuergerät 2 zumindest in Grenzen verschwenkt werden kann. Mindestens ein Bereich des Haltearms 30 ist biegsam gestaltet. Das können z. B. die Kniebereiche 30.1 und/oder 30.2 sein.

Innerhalb des Haltearms 30 verläuft ein Bündel von Stromleitungen 5, deren eine Enden an das Steuergerät 2 angeschlossen sind, und über das Steuerpult 2.1 angesteuert werden. Die Stromleitungen 5 werden in dem Haltearm 30 zur Modulplatte 31 geführt und werden dort vereinzelt. Ihre dortigen Enden sind jeweils an ein Lasermodul 33 angeschlossen, die ihrerseits über hängende Lichleittfasern 34 mit Lasernadeln 35 verbunden sind. Die Lichtleitfasern 34 sind jeweils durch eine nicht dargestellte Umhüllung geschützt

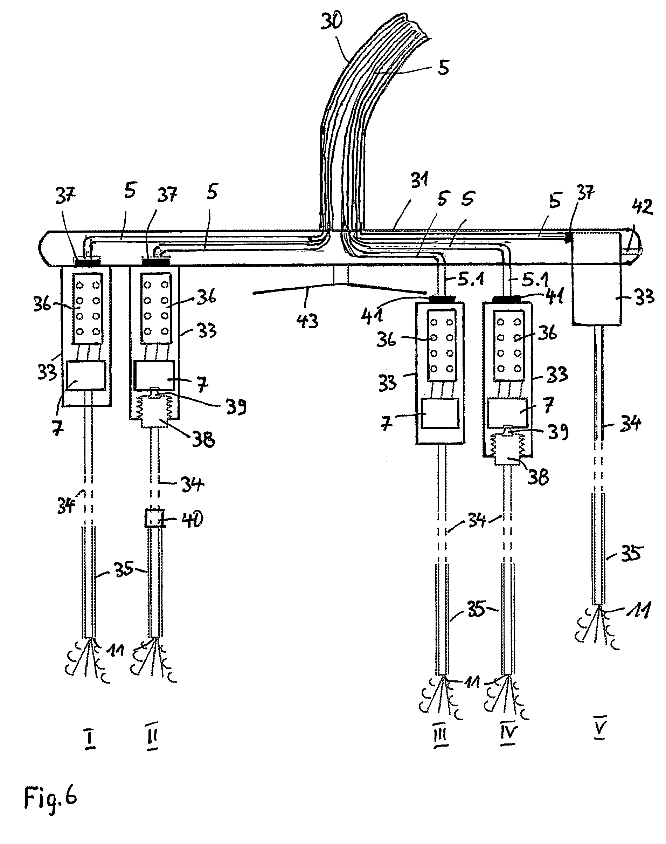

Der Aufbau und die Anordnung der Lasermodule 33 an der Modulplatte 31 wird nachstehend anhand der Figur 6 näher erläutert.

In Figur 6 sind exemplarisch verschiedene Möglichkeiten I bis V einer Verbindung zwischen den Stromleitungen 5 und den Lasermodulen 33 bzw. zwischen den Lasermodulen 33 und den Lichtleitfasern 34 dargestellt. In allen Fällen bestehen die Lasermodule 33 aus einer Laserdiode 7 und einem Treiber-Chip 36. In den Fällen I und II sind die Lasermodule 33 jeweils über in der Modulplatte 31 vorgesehene Steckkontakte 37 mit den ihnen zugeordneten Stromleitungen 5 verbunden. Zum Austausch der Lasermodule 33 werden diese einfach aus der Modulplatte 31 herausgezogen und neue Lasermodule 33 werden eingesteckt. Auf diese Weise können Reparaturen oder auch ein Wechsel der Lasermodule 33 mit verschiedenen Wellenlängen und Diodenstärken auf einfache Weise durch den Behandler selbst vorgenommen werden. Im Falle I werden mit dem Lasermodul 33 allerdings auch die mit diesem verbundene Lichtleitfaser 34 und die Lasernadel 35 ausgewechselt, da die Lichtleitfaser 34 in das Lasermodul 33 eingeklebt ist. Im Fall II wird dies dadurch vermieden, dass zwischen dem Lasermodul 33 und der Lichtleitfaser 34 ein optischer Stecker 38 vorgesehen ist. Zur Verbindung mit dem Lasermodul 33 wird die Lichtleitfaser 34 einfach in diesen Stecker 38 eingesteckt. Die Kopplung mit der Laserdiode 7 erfolgt über ein optisches Koppelelement 39. Im Fall II können also die Lasermodule 33 und die Lichtleitfasern 34 separat ausgewechselt werden. Dieses System kann noch weiter gesteigert werden, in dem zwischen der Lichtleitfaser 34 und der Lasernadel 35 eine weitere Steckverbindung 40 mit optischer Einkopplung vorgesehen ist. In diesem Fall kann auch die Lasernadel 35 separat ausgetauscht werden. Auch die Laserdiode 7 kann jeweils separat in das aus Metall bestehende Gehäuse des Lasermoduls 33 eingeschraubt sein, so dass diese bei isoliertem Defekt auch allein ausgewechselt werden kann. Durch die Einschraubung der Laserdiode 7 in das Gehäuse des Lasermoduls 33 und engen Kontakt mit dem Gehäusemetall findet eine gute Abfuhr der von den Laserdioden 7 erzeugten Wärme statt.

Die Fälle III und IV unterscheiden sich von den Fällen I bzw. II dadurch, dass die Lasermodule 33 nicht in die Modulplatte 31 eingesteckt sind, sondern dass die Elektroleitungen 5 ein kurzes Stück 5.1 aus der Modulplatte 31 nach unten herausgeführt sind. Zwischen den Enden der Stromleitungen 5 und den Lasermodulen 33 sind ebenfalls wieder Steckverbindungen 41 vorgesehen.

Im Fall V sind die Lasermodule 33 in eine entsprechende Öffnung in der Modulplatte 31 eingelassen und dort mittels Madenschrauben 42 befestigt. Die Modulplatte 31 ist in diesem Falle als Metallblock ausgeführt. Durch den Kontakt der Lasermodule 33 mit diesem Metallblock erfolgt eine Wärmeabführung aus dem Lasermodul 33 in den Metallblock.

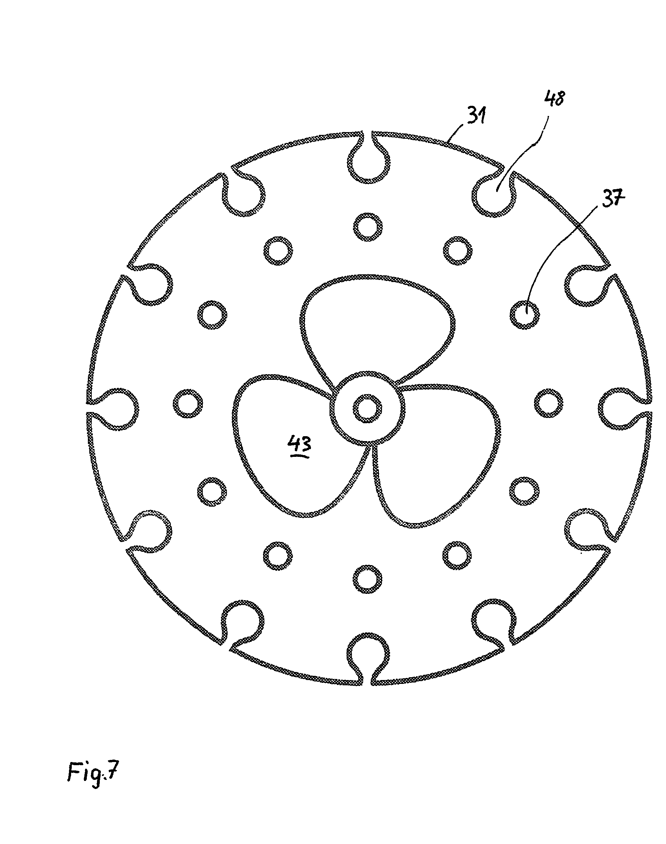

Zusätzlich zu dieser Kühlung oder alternativ dazu kann im zentralen Bereich der Modulplatte 31 ein Lüfter 43 vorgesehen sein. Die Lasermodule 33 sind dann im peripheren Bereich der Modulplatte 31 angeordnet, umringen also den Lüfter 43. Diese Ausbildung geht am besten aus der Darstellung gemäß Figur 7 hervor. Der Lüfter 43 sorgt für eine intensive Kühlung der sich stark erwärmenden Lasermodule 33, so dass deren Lebensdauer wesentlich erhöht wird.

Aus der Darstellung gemäß Figur 7 geht hervor, dass am Rand der Modulplatte 31 Ausnehmungen 48 vorgesehen sind. Diese dienen dem Einhängen der Lasernadeln 35, wenn diese nicht gebraucht werden.

In allen Fällen I bis V können die Lasernadeln 35 als kombinierte Laserelektronadeln ausgeführt sein. Hier sorgen die vom Steuergerät 2 zu den Laserelektronadeln in der Umhüllung der Lichtleitffasern geführten Stromleitungen für eine zusätzlichen Schutz der Lichtleitfasern gegen Bruch.

Zur Befestigung der Lasernadeln 35 auf der Haut 46 eines Patienten sind Einmalhülsen 44 aus preiswertem Material, wie zum Beispiel aus Pappe oder Kunststoff, vorgesehen. Diese besitzen einen Schaft 44.1, der auf die Lasernadel 35 aufschiebbar ist und an dieser durch Reibung festhält. Der Schaft 44.1 geht in eine breitere Basis 44.2 über, die der Herstellung einer Klebverbindung mit der Haut 46 eines Patienten dient.

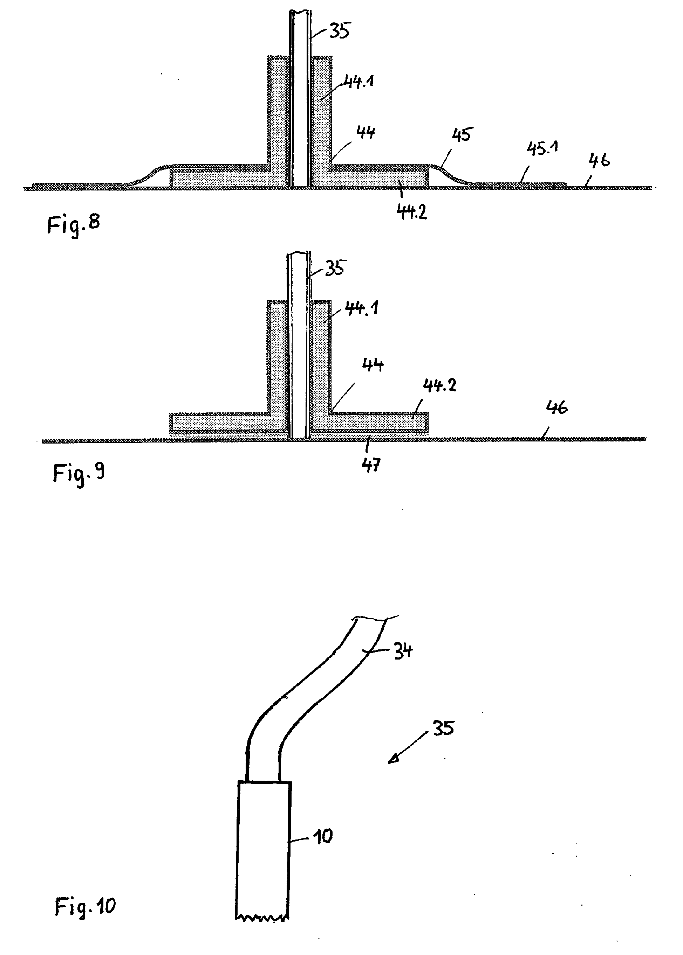

Im Falle des Ausführungsbeispiels gemäß Figur 8 wird die Klebverbindung durch ein Lochpflaster 45 hergestellt. Dieses ist über den Schaft 44.1 geschoben und liegt auf der Oberseite der Basis 44.2 auf. Der die Basis 44.2 der Einmalhülse 44 überragende Bereich 45.1 des Lochpflasters 45 dient dem Aufkleben auf der menschlichen Haut 46. Einmalhülse 44 und Lochpflaster 45 können auf einer Abziehfolie als schon fertige Einheit zur Verfügung gestellt werden.

Im Ausführungsbeispiel gemäß Figur 9 ist auf der Unterseite der Basis 44.2 ein Klebering 47 vorgesehen, über den die Klebverbindung mit der menschlichen Haut 46 vorgenommen wird. Auch die Einmalhülse 44 und der Klebering 47 können als Einheit auf einer Abziehfolie zur Verfügung gestellt werden.

In einer weiteren Ausführungsform der Erfindung ist das untere Ende der die Lasernadel 9, 35 umhüllenden Kunststoffhülse 10 mit kleinen Zähnen versehen. Dadurch wird der Halt der Lasernadel 9 an schwierigen Körperteilen, wie z. B. an einem Ohr, verbessert.

## Patentansprüche

1. Vorrichtung zur Akupunktur mittels Laserstrahlung mit folgenden Merkmalen: einem Steuergerät (2), welches mehrere Ausgänge (3) für Stromleitungen (5) aufweist, mehreren Stromleitungen (5), welche an die Ausgänge (3) des Steuergeräts (2) angeschlossen sind, mehreren Laserdioden (7), welche jeweils an die Stromleitungen (5) angeschlossen sind, **dadurch gekennzeichnet, dass** die Vorrichtung zusätzliche Merkmale umfasst: mehrere Lasernadeln (9, 35), welche mit den Laserdioden (7) derart verbunden sind, dass die von den Laserdioden (7) ausgesandte Laserstrahlung in die Lasernadeln (9, 35) eingekoppelt wird, und welche dafür vorgesehen sind, in ihrem Ausgangsbereich (11) mit dem Körper eines Patienten in Kontakt zu treten, wobei die Stromleitungen (5) im Bündel in oder an einem Haltearm (30) von dem Steuergerät (2) zu einer am Ende des Haltearms (30) angeordneten Modulplatte (31) geführt sind, in der eine Vereinzelung der Stromleitungen (5) des Bündels erfolgt, wobei an die Enden der Stromleitungen (5) in oder an der Modulplatte (31) jeweils ein ein Treiberchip (36) und eine Laserdiode (7) aufweisendes Lasermodul (33) angeschlossen ist, wobei die Lasermodule (33) jeweils über hängende, durch Umhüllungen geschützte Lichtleitfasern (34) mit den Lasernadeln (35) verbunden sind.

2. Vorrichtung nach Anspruch 1, **dadurch gekennzeichnet, dass** die Lasermodule (33) im peripheren Bereich der Modulplatte (31) angeordnet sind und im zentralen Bereich ein Lüfter (43) zur Kühlung der Lasermodule (33) angeordnet ist.

3. Vorrichtung nach Anspruch 1 oder 2, **dadurch gekennzeichnet, dass** am Rand der Modulplatte (31) Ausnehmungen (48) zum Einhängen der Lasernadeln (35) vorgesehen sind.

4. Vorrichtung nach einem der Ansprüche 1 bis 3 **dadurch gekennzeichnet, dass** die Enden der Stromleitungen (5) aus der Modulplatte (31) ein kurzes Stück (5.1) herausgeführt sind.

5. Vorrichtung nach einem der Ansprüche 1 bis 4, **dadurch gekennzeichnet, dass** der Haltearm (30) schwenkbar und/oder zumindest in einem Bereich seiner Länge (30.1, 30.2) biegbar ist.

6. Vorrichtung nach einem der vorstehenden Ansprüche **dadurch gekennzeichnet, dass** die Stromleitungen (5) mittels jeweiliger Steckverbindungen (6, 37, 41) mit den Laserdioden (7) bzw. Lasermodulen (33) verbunden sind.

7. Vorrichtung nach einem der vorstehenden Ansprüche, **dadurch gekennzeichnet, dass** die Stromleitungen (5) mittels jeweiliger Steckverbindungen (4) mit dem Steuergerät (2) verbunden sind.

8. Vorrichtung nach einem der vorstehenden Ansprüche **dadurch gekennzeichnet, dass** die Lasernadeln (9, 35) und/oder ein Kern der Lasernadeln (9, 35) sich von der Laserdiode (7) zu ihrem Ausgangsbereich (11) verjüngen.

9. Vorrichtung nach einem der vorstehenden Ansprüche **dadurch gekennzeichnet, dass** die Laserstrahlung mittels des Steuergeräts (2) frequenzmodulierbar ist.

10. Vorrichtung nach einem der vorstehenden Ansprüche, **dadurch gekennzeichnet, dass** die einzelnen Laserdioden (7) unterschiedliche Wellenlängen und/oder unterschiedliche Leistungen aufweisen.

11. Vorrichtung nach einem der vorstehenden Ansprüche **dadurch gekennzeichnet dass** das Steuergerät (2) ein Photonleter (13) zum Messen der Intensität der Laserstrahlung in dem Ausgangsbereich (11) der Lasernadel (9, 35) aufweist.

12. Vorrichtung nach einem der vorstehenden Ansprüche, **dadurch gekennzeichnet, dass** das Steuergerät (2) mittels einer Batterie betreibbar ist.

13. Vorrichtung nach einem der vorstehenden Ansprüche, **dadurch gekennzeichnet, dass** die Lasernadeln (9, 35) mit Kunststoffhülsen (10) überzogen sind.

14. Vorrichtung nach Anspruch 15, **dadurch gekennzeichnet, dass** das untere Ende der Kunststoffhülse (10) mit kleinen Zähnen versehen ist.

15. Vorrichtung nach einem der vorstehenden Ansprüche, **dadurch gekennzeichnet, dass** auf die Lasernadel (9, 35) eine Einmalhülse (44) aus preiswertem Material, wie z.B. Pappe oder Kunststoff, mit einem Schaft (44.1) aufschiebbar ist, der durch Reibung an der Lasernadel (9,35) hält und in eine breitere Basis (44.2) zur Herstellung einer Klebverbindung mit der Haut (46) eines Patienten übergeht.

16. Vorrichtung nach Anspruch 15, **dadurch gekennzeichnet, dass** auf der Unterseite der Basis (44) eine Klebschicht (47) angeordnet ist.

17. Vorrichtung nach Anspruch 15, **dadurch gekennzeichnet, dass** ein Lochpflaster (45) über den Schaft (44.1) schiebbar ist, dessen Klebschicht die Basis (44.2) seitlich überragt.

18. Vorrichtung nach einem der vorstehenden Ansprüche, **dadurch gekennzeichnet, dass** die Lasernadel (9, 35) von einer stromleitenden Hülse (14) umgeben ist, welche von dem Steuergerät (2) mit Strom versorgt wird.

## Claims

1. An acupuncture device using laser radiation, having the following features: a control device (2), which comprises a plurality of output terminals (3) for current leads (5), a plurality of current leads (5), which are connected to the output terminals (3) of the control device (2), a plurality of laser diodes (7), which are in each case connected to the current leads (5), **characterised in that** the device comprises additional features: a plurality of laser needles (9, 35), which are connected to the laser diodes (7) in such a way that the laser radiation emitted by the laser diodes is coupled into the laser needles (9, 35) and which are provided for the purpose of coming into contact with the body of a patient in their output region (11), the current leads (5) being guided in a bundle in or on a support arm (30) of the control device (2) to a module plate (31) arranged at the end of the support arm (30), in which the bundled current leads (5) are separated out, a laser module (33) comprising a driver chip (36) and a laser diode (7) being in each case connected to the ends of the current leads (5) in or on the module plate (31), the laser modules (33) being connected to the laser needles (35) in each case by means of optical fibres (34) hanging down and protected by sheathing.

2. A device according to claim 1, **characterised in that** the laser modules (33) are arranged in the peripheral region of the module plate (31), a fan (43) being arranged in the central region for cooling the laser modules (33).

3. A device according to claim 1 or claim 2, **characterised in that** recesses (48) for suspension of the laser needles (35) are provided at the edge of the module plate (31).

4. A device according to any one of claims 1 to 3, **characterised in that** the ends of the current leads (5) extend a short distance (5.1) out of the module plate (31).

5. A device according to any one of claims 1 to 4, **characterised in that** the support arm (30) is swivellable and/or bendable at least in one place over its length (30.1, 30.2).

6. A device according to any one of the preceding claims, **characterised in that** the current leads (5) are connected to the laser diodes (7) or laser modules (33) by means of respective plug-in connections (6, 37, 41).

7. A device according to any one of the preceding claims, **characterised in that** the current leads (5) are connected to the control device (2) by means of respective plug-in connections (4).

8. A device according to any one of the preceding claims, **characterised in that** the laser needles (9, 35) and/or the cores of the laser needles (9, 35) taper from the laser diode (7) to their output region (11).

9. A device according to any one of the preceding claims, **characterised in that** the laser radiation may be frequency-modulated by means of the control device (2).

10. A device according to any one of the preceding claims, **characterised in that** the individual laser diodes (7) have different wavelengths and/or different powers.

11. A device according to any one of the preceding claims, **characterised in that** the control device (2) comprises a photometer (13) for measuring the intensity of the laser radiation in the output region (11) of the laser needles (9, 35).

12. A device according to any one of the preceding claims, **characterised in that** the control device (2) may be battery-operated.

13. A device according to any one of the preceding claims, **characterised in that** the laser needles (9, 35) are covered with plastics sleeves (10).

14. A device according to claim 15, **characterised in that** the lower end of the plastics sleeve (10) is provided with small teeth.

15. A device according to any one of the preceding claims, **characterised in that** a disposable sleeve (44) of inexpensive material, such as for example paperboard or plastics, may be pushed onto the laser needle (9, 35) with a shank (44.1), which is retained on the laser needle (9, 35) by friction and develops into a wider base (44.2) for producing an adhesive bond with the skin (46) of a patient.

16. A device according to claim 15, **characterised in that** an adhesive layer (47) is arranged on the underside of the base (44).

17. A device according to claim 15, **characterised in that** a perforated plaster (45) may be pushed over the shank (44.1), its adhesive layer projecting laterally beyond the base (44.2).

18. A device according to any one of the preceding claims, **characterised in that** the laser needle (9, 35) is surrounded by a current-conducting sleeve (14), which is supplied with current by the control device (2).

## Revendications

1. Dispositif d'acupuncture au moyen d'un rayonnement laser, dont les caractéristiques sont les suivantes : un appareil de commande (2), qui présente plusieurs sorties (3) pour des conduites de courant (5), plusieurs conduites de courant (5), qui sont raccordées aux sorties (3) de l'appareil de commande (2), plusieurs diodes (7) qui sont respectivement raccordées aux conduites de courant (5), **caractérisé en ce que** le dispositif comprend les caractéristiques supplémentaires suivantes : plusieurs aiguilles laser (9, 35), qui sont reliées aux diodes laser (7) de manière que le rayonnement laser émis par les diodes laser soit injecté dans les aiguilles laser (9, 35), et qui sont destinées à entrer en contact, au niveau de leur zone de sortie (11), avec le corps du patient, les conduites de courant (5) regroupées étant guidées dans ou sur un bras support (30) depuis l'appareil de commande (2) jusqu'à une plaque module (31), disposée à l'extrémité du bras support (30), dans laquelle a lieu une séparation des conduites de courant (5) regroupées, un module laser (33), qui présente respectivement un circuit intégré driver et une diode laser (7), étant raccordé aux extrémités des conduites de courant (5), dans ou sur la plaque module (31), les modules laser (33) étant respectivement reliés aux aiguilles laser (35) par l'intermédiaire de fibres optiques (34) protégées par des gaines.

2. Dispositif suivant la revendication 1, **caractérisé en ce que** les modules laser (33) sont disposés dans la zone périphérique de la plaque module (31) et **en ce qu'**un aérateur (43) est disposé dans la zone centrale pour le refroidissement des modules laser.

3. Dispositif suivant la revendication 1 ou 2, **caractérisé en ce que** des creux (48) permettant d'y accrocher les aiguilles laser sont prévus (35) sur le bord de la plaque module (31).

4. Dispositif suivant une des revendications 1 à 3, **caractérisé en ce qu'**une courte partie (5.1) des extrémités des conduites de courant (5) font saillie de la plaque module (31).

5. Dispositif suivant une des revendications 1 à 4, **caractérisé en ce que** le bras support (30) est orientable et/ou pliable au moins dans une zone de sa longueur (30.1, 30.2).

6. Dispositif suivant une des revendications précédentes, **caractérisé en ce que** les conduites de courant (5) sont reliées aux diodes laser (7), c'est-à-dire aux modules laser (33), au moyen de connecteurs respectifs (6, 37, 41).

7. Dispositif suivant une des revendications précédentes, **caractérisé en ce que** les conduites de courant (5) sont reliées à l'appareil de commande (2) au moyen de connecteurs respectifs (4).

8. Dispositif suivant une des revendications précédentes, **caractérisé en ce que** les aiguilles laser (9, 35) et/ou un noyau des aiguilles laser (9, 35) diminuent depuis la diode laser (7) jusqu'à leur zone de sortie (11).

9. Dispositif suivant une des revendications précédentes, **caractérisé en ce que** la fréquence du rayonnement laser peut être modulée au moyen de l'appareil de commande (2).

10. Dispositif suivant une des revendications précédentes, **caractérisé en ce que** chaque diode laser (7) présente une longueur d'onde et/ou une puissance différente.

11. Dispositif suivant une des revendications précédentes, **caractérisé en ce que** l'appareil de commande (2) présente un photoconducteur (13) pour mesurer l'intensité du rayonnement laser dans la zone de sortie (11) de l'aiguille laser (9, 35).

12. Dispositif suivant une des revendications précédentes, **caractérisé en ce que** l'appareil de commande (2) fonctionne au moyen d'une pile.

13. Dispositif suivant une des revendications précédentes, **caractérisé en ce que** les aiguilles laser (9, 35) sont revêtues de douilles en matière artificielle (10).

14. Dispositif suivant la revendication 15, **caractérisé en ce que** l'extrémité inférieure de la douille en matière artificielle (10) est pourvue de petites dents.

15. Dispositif suivant une des revendications précédentes, **caractérisé en ce que** sur l'aiguille laser (9, 35) peut être poussée une douille à usage unique (44) en une matière peu coûteuse, par exemple en carton ou en matière artificielle, avec une tige (44.1) qui tient par friction sur l'aiguille laser (9,35) et qui se transforme en une base plus large (44.2) pour réaliser une liaison adhésive avec la peau (46) d'un patient.

16. Dispositif suivant la revendication 15, **caractérisé en ce qu'**une couche adhésive (47) est disposée sur la face inférieure de la base (44).

17. Dispositif suivant la revendication 15, **caractérisé en ce qu'**un pansement perforé (45), dont la couche adhésive dépasse la base sur les côtés, peut être poussé sur la tige (44.1).

18. Dispositif suivant une des revendications précédentes, **caractérisé en ce que** l'aiguille laser (9, 35) est entourée d'une douille conductrice (14), qui est alimentée en courant par l'appareil de commande (2).
